# EUROPEAN PATENT SPECIFICATION

(11) **EP 0 836 707 B1**
(45) Date of publication and mention of the grant of the patent: **18.01.2006**
(21) Application number: 96918330.0
(22) Date of filing: 07.06.1996
(51) Int. Cl.: G01N 33/569, C07K 14/15

(54) **IMPROVED HAPTEN-PEPTIDE CONJUGATES TO DETECT ANTI-HIV-1 OR ANTI-HIV-2 ANTIBODIES**
VERBESSERTE HAPTEN-PEPTIDKONJUGATEN ZUM NACHWEIS VON ANTI-HIV-1 ODER ANTI-HIV-2 ANTIKÖRPERN
CONJUGUES HAPTENE/PEPTIDE AMELIORES UTILISES POUR DETECTER DES ANTICORPS ANTI-VIH1 OU ANTI-VIH2

(30) Priority: 07.06.1995 US 486657
(43) Date of publication of application: 22.04.1998
(73) Proprietor: ABBOTT LABORATORIES, Abbott Park, Illinois 60064-3500 (US)
(72) Inventor: DAGHFAL, David, J., Aurora, IL 72477 (US); COLPITTS, Tracey, L., Round Lake, IL 60073 (US); CHANG, Chi-Deu, Green Oaks, IL 60048 (US); MERCHANT, Barbara, T., Wilmette, IL 60091 (US); SZE, Isaac, S.-Y., Gurnee, IL 60031 (US); JAFFE, Keeve, Trevor, WI 53179 (US); BRIDON, Dominique, P., Morton Grove, IL 60053 (US)
(74) Representative: Modiano, Micaela Nadia
(86) International application number: PCT/US1996/009507
(87) International publication number: WO 1996/041187

(56) References cited:
- WO-A-88/10316
- WO-A-90/08957
- FEMS MICROBIOLOGY IMMUNOLOGY, vol. 89, 1991, AMSTERDAM NL, pages 57-64, XP002017275 P.M. RICHALET-S¹CORDEL ET AL.: "A new capture test using conjugated peptides for the detection of HIV antibodies."

## Description

### Background of the Invention

This invention relates generally to hapten-peptide conjugates and more particularly, relates to an improved immunoassay for detecting HIV-1 and HIV-2 using hapten-peptide conjugates.

Haprens are used in various immunoassays to increase or amplify the signal generated by a signal generating compound used to detect an analyte in a test sample, thus increasing the sensitivity of the assay. The term "hapten" refers to a partial antigen or non-protein binding member which is capable of binding to an antibody, but which is not capable of eliciting antibody formation unless coupled to a carrier protein. Examples of haptens include molecules such as biotin or and-biotin and avidin or biotin, and the like.

Biotinylated peptides, for example, have been prepared by biotinylating an antigen or a peptide in solution with a selected molar input of biotinylation reagent and removing free biotin from the crude probe by dialysis. There are, however, numerous problems associated with this process of biotinylation. The biotinylated peptides produced by this process typically are a mixture of peptides tagged with various and different numbers of biotin moieties. Further, because the biotinylation is random on the peptide, it is likely that some peptide molecules in the solution are not biotinylated at any position. Thus, peptides made with the solution biotinylation process are likely to perform inconsistently in different preparations. Moreover, when the biotinylated peptide is an antigen to be used in an immunoassay, the solution biotinylation process usually results in biotinylation of amino acid residues within the antigen epitope, which then interferes with binding between the antigen and antibody. In addition, since biotin attaches randomly on the peptide molecule, there is no control over where the biotinylation occurs on the peptide, and the amount of biotin present on the pepddes in preparation of biotinylated peptides is unknown and uncontrollable.

WO90/08957 discloses a method for detection for screening and diagnostic purposes of antibodies in a body fluid by means of an antibody-binding protein covalently bound to a porous matrix trapped within a transparent column. According to this document, a test fluid containing antibody is contacted with the protein-bound matrix whereby the protein immobilizes any antibody present in the test fluid. A randomly biotinylated antigen of interest is contacted with and binds to the immobilized antibody; avidin covalently linked to an enzyme is contacted with and binds to the immobilized biotinylated immobilized complex, which is then contacted with a substrate solution so as to have a detectable product. Similarly, Voller et al. disclose a biotin-avidin sandwich test in which the sample is incubated, a biotin-labeled antibody is added and then an avidin-labeled enzyme is added.

US 4,957,737 discloses synthetic peptides which can be used as reagents in immunoassays for detection of AIDS antibodies. In this document, the peptide sequence that is used as probe is radiolabeled at the terminal cysteine residue.

Campbell et al. disclose the use of biotynilated growth hormone releasing factor analogs; in their publication, they indicate that GFR bioactivity is relatively intolerant to N-terminal modifications, and thus postulate that GRF requires biotinylation at a site distant from the bioactive core in order to retain its growth hormone releasing activity.

There is a need therefore for improved hapten-peptide conjugates in which the hapten, preferably biotin, is attached at known and pre-detennined specific location or locations on the peptide molecule.

### Summary of the Invention

The present invention provides improved immunoassays for detecting antibodies against HIV-1 and HIV-2 that may be present in a test sample. Immunoassays for HIV-1 or HIV-2 that are improved as taught herein, generally comprise contacting a test sample with a capture reagent for an antibody analyte, wherein said capture reagent comprises a binding pair member specific for the antibody analyte of interest attached to a solid phase, to thereby form a first mixture. This first mixture is incubated for a time and under conditions sufficient to form capture reagent / analyte complexes. These complexes then are contacted with a hapten-peptide conjugate comprising a hapten attached to a peptide specific for the analyte member of the complex to form a second mixture. This second mixture is incubated to form capture reagent / analyte / hapten-peptide conjugate complexes. Then, a wash step preferably is performed on the capture reagent / analyte / hapten-peptide conjugate complexes to separate any uncomplexed hapten-peptide conjugate from the capture reagent / analyte / hapten-peptide conjugate complexes. Next, an indicator reagent comprising a member of a binding pair specific for the hapten which is labeled with a signal generating compound capable of generating a measurable signal is contacted with the capture reagent / analyte / hapten-peptide conjugate complexes to form a third mixture. This third mixture is incubated for a time and under conditions sufficient to form capture reagent / analyte / hapten-peptide conjugate / indicator reagent complexes. The presence, if any, of the analyte is determined by detecting the measurable signal generated by the signal generating compound. The improvement to the assay comprises contacting the capture reagent / analyte complexes with a hapten-peptide conjugate comprising an amino acyl residue sequence designated herein as SEQUENCE LD. NOS.1-5 which is labeled with at least one and preferably two N-terminal haptens at the a and/or ε position(s).

Also provided is a composition useful for detecting anti HIV-1 or anti-HIV-2 antibodies that may be present in a test sample. The composition comprises a substantially pure N-terminal haptenated peptide which is selected from any of the sequences designated herein as SEQUENCE LD. NOS.1-5. In addition, the invention provides a test kit comprising at least one hapten-peptide conjugate reagent. The hapten-peptide conjugate reagent generally comprises a substantially pure N-terminal haptenated peptide which is selected from any of the sequences designated herein as SEQUENCE I.D. NOS.1-5 dissolved or otherwise suspended in an appropriate buffer.

### Brief Description of the Drawinas

FIGURE 1 is a schematic outline of a biotinylation process.
FIGURE 2 shows a plot of S/N ratios versus peptide concentration dose responses of a Cameroon sample to biotinylated HIV-1 gp41 antigens.
FIGURE 3 shows a semi-log plot of counts v. peptide concentration dose responses of HIV samples to SEQUENCE LD. NO. 6.

### Detailed Description of the Invention

Unless otherwise indicated, the following terms have the following meanings:

The term "synthetic peptide" as used herein means a polymeric form of amino acids of any length which may be chemically synthesized by methods well-known to the routineer. These synthetic peptides are useful in various applications.

A synthetic peptide or "antigen" is "immunologically reactive" with an antibody when it binds to an antibody due to antibody recognition of a specific epitope contained within the peptide. Immunological reactivity may be determined by antibody binding, more particularly by the kinetics of antibody binding, and/or by competition in binding using as competitor(s) a known peptide(s) containing an epitope against which the antibody is directed. The methods for determining whether a peptide is immunologically reactive with an antibody are known in the art.

The term "individual" as used herein refers to vertebrates, particularly members of the mammalian species and includes but is not limited to domestic animals, sports animals, primates and humans; more particularly the term refers to primates/simians and humans.

The term "analyte containing body component" or "test sample" refers to a component of an individual's body which is the source of the antibody analyte of interest. These components are well known in the art. These test samples include biological samples which can be tested by the methods of the present invention described herein and include human and animal body fluids such as whole blood, serum, plasma, cerebrospinal fluid, urine, lymph fluids, ascites fluid and various external secretions of the respiratory, intestinal and genitoutinary tracts, tears, saliva, milk, white blood cells, myelomas and the like; biological fluids such as cell culture supernatants; fixed tissue specimens; and fixed cell specimens or any other body constituents.

The improved hapten-peptide conjugates disclosed herein can be used to develop unique and improved assays as described herein to detect or confirm the presence of anti HIV-1 or anti HIV-2 antibody. The hapten-peptide conjuates also may be used in combination with other hapten conjugates comprising a hapten and, for example, native viral antigens and/or recombinant proteins.

"Analyte," as used herein, is the substance to be detected which may be present in the test sample. The analyte can be any substance for which there exists a naturally occurring specific binding member (such as, an antibody), or for which a specific binding member can be prepared. Thus, an analyte is a substance that can bind to one or more specific binding members in an assay. "Analyte" also includes any antigenic substances, haptens, antibodies, and combinations thereof. As a member of a specific binding pair, the analyte can be detected by means of naturally occurring specific binding partners (pairs) such as the use of intrinsic factor protein as a member of a specific binding pair for the determination of Vitamin B12, the use of folate-binding protein to determine folic acid, or the use of a lectin as a member of a specific binding pair for the determination of a carbohydrate. The analyte also can include a protein, a peptide, an amino acid, a nucleotide target, and the like.

The present invention provides assays which utilize specific binding members. A "specific binding member," as used herein, is a member of a specific binding pair. That is, two different molecules where one of the molecules through chemical or physical means specifically binds to the second molecule. Therefore, in addition to antigen and antibody specific binding pairs of common immunoassays, other specific binding pairs can include biotin and avidin, carbohydrates and lectins, complementary nucleotide sequences, effector and receptor molecules, cofactors and enzymes, enzyme inhibitors and enzymes, and the like. Furthermore, specific binding pairs can include members that are analogs of the original specific binding members, for example, an analyte-analog. The specific binding pair member can include a protein, a peptide, an amino acid, a nucleotide target, and the like. Furthermore, specific binding pairs can include members that are analogs of the original specific binding members, for example, an analyte-analog. Immunoreactive specific binding members include antigens, antigen fragments, antibodies and antibody fragments, both monoclonal and polyclonal, and complexes thereof, including those formed by recombinant DNA molecules. The term "hapten", as used herein, refers to a partial antigen or non-protein binding member which is capable of binding to an antibody, but which is not capable of eliciting antibody formation unless coupled to a carrier protein.

A "capture reagent", as used herein, refers to an unlabeled specific binding member which is specific either for the analyte as in a sandwich assay, for the indicator reagent or analyte as in a competitive assay, or for an ancillary specific binding member, which itself is specific for the analyte, as in an indirect assay. The capture reagent can be directly or indirectly bound to a solid phase material before the performance of the assay or during the performance of the assay, thereby enabling the separation of immobilized complexes from the test sample.

The "solid phase" is not critical and may be any variety of materials which may be selected by one skilled in the art without undue experimentation. The term "solid phase" is used in a broad sense and refers to any material which is insoluble, or may be made insoluble by a subsequent reaction. Thus, porous or nonporous materials, latex or polystyrene particles, magnetic or non-magnetic microparticles, beads, membranes, plastic tubes, walls of microtiter wells and tanned sheep red blood cells are all suitable examples. The size, dimensions, and shape of the solid phase generally are not critical in practicing the methods disclosed herein.

Suitable methods for immobilizing antigens on solid phases include ionic, hydrophobic, covalent interactions and the like; a variety of methodologies may be applied relative to the application of useful solid phases. The capture reagent may be bound either passively or actively on the solid phase. Passive coating, as used herein, means non-covalent bonding or non-covalent attachment between the capture reagent and the solid phase. Active coating means effecting a covalent bond between the capture reagent and the solid support. Generally, such a covalent bond is formed either by the carboxy or the amino terminal end of an antigen or by a free carboxy or amino group within the protein binding to an appropriate functional group on the surface of the solid phase. Such solid phases having such functional groups are termed derivatized. Active coating also can encompass the use of linker compounds to effect the formation of a covalent bond between the antigen and the solid support. The linking agent can be incorporated as part of, or derivatized onto, the solid phase before the capture reagent, usually an antigen, is added. The use of heterobifunctional such as sulfo-SMCC (sulfosuccinimidyl 4-(N-maleimidomethyl)cyclohexane-1-carboxylate) or sulfo-SIAB (sulfosuccinmidyl(4-iodoacetyl)aminobenzoate) or homobifunctional compounds such as sulfo-DST (disufosuccinimidyl tartarate) or sulfo-EGS (ethylene glycolbis[sulfosuccinimidyl-succinate]) allows more specific/site directed covalent bonding to occur. The addition of the linker compound also can include a spacer arm which would allow the antigen to more freely interact with antibodies while attached to the solid phase. Linker group chemistry is well known to the skilled artisan.

A suitable "indicator reagent" comprises a signal generating compound (label) which is capable of generating a measurable signal detectable by external means conjugated (attached) to a specific binding member. The various "signal generating compounds" (labels) contemplated include chromogens, catalysts such as enzymes, luminescent compounds such as fluorescein and rhodamine, chenuluminescent compounds such as luminol, dioxetanes, acridinium compounds and phenanthridinium compounds, radioactive elements and direct visual labels. Examples of enzymes include alkaline phosphatase, horseradish peroxidase, beta-galactosidase, and the like. The selection of a particular label is not critical, but it will be capable of producing a signal either by itself or in conjunction with one or more additional substances.

An analyte which may be present in a test sample can be detectable in assays by use of a hapten-peptide conjugate disclosed herein. Also, as one of the reagents of the assay, different synthetic peptides capable of identifying and specifically binding to different epitopes of an analyte such as a virus or bacteria can be used in assay formats. Haptenated peptides of the present invention can be used for binding, linking or signal amplification purposes which are well-known in the art.

According to the present invention, an improvement to an assay designed to detect the presence of anti HIV-1 or anti HIV-2 antibody analyte is provided. The assays which are improved upon as taught herein typically are performed as follows. A test sample is contacted with a capture reagent of the analyte, wherein said capture reagent comprises a binding pair member specific for the antibody analyte of interest attached to a solid phase, to thereby form a first mixture. Since the analyte is an antibody, the capture reagent can be a recombinant antigen, a synthetic peptide or a lysate preparation which specifically binds to the analyte. The first mixture is incubated for a time and under conditions sufficient to form capture reagent/ analyte complexes. These so-formed complexes then are contacted with a hapten-peptide conjugate to form a second mixture. This second mixture is incubated for a time and under sufficient conditions to form capture reagent / analyte / hapten-peptide conjugate complexes. Then, a wash step preferably is performed. Next, an indicator reagent comprising a member of a binding pair specific for the hapten which is labeled with a signal generating compound capable of generating a measurable signal is contacted with the capture reagent / analyte / hapten-peptide conjugate complexes to form a third mixture. This third mixture is incubated for a time and under conditions sufficient to form capture reagent / analyte / hapten-peptide conjugate / indicator reagent complexes. The presence of the analyte, if any, is determined by detecting the signal generated by the signal generating compound. It is possible to quantitate the amount of analyte present by also assaying positive calibrators which contain known concentrations of analyte and negative calibrators, plotting the results of the measured signal obtained with these calibrators against the known concentrations, and reading the results of the test samples off of the plots.

The improvement provided herein comprises contacting the capture reagent / analyte complexes with at least one of the peptides designated herein as SEQUENCE ID NO. 1-5 which has been haptenated at the N terminal α and/or ε position(s). Preferably, the hapten is biotin. Also provided herein is a composition useful in detecting anti HIV-1 or anti HIV-2 antibody. The composition generally comprises a substantially pure solution of at least one N-terminal haptenated peptide such as those designated herein as SEQUENCE LD. NOS. 1-5. As used herein a "substantially pure solution" as used herein will be understood to mean that the concentration of peptides haptenated at non-N-terminal positions in the solution will not bind the antibody anaiyte in amounts that effect the assay results. Those skilled in the art will recognize suitable buffers for suspending or dissolving the hapten-peptide conjugates provided herein.

A substantially pure solution hapten-peptide conjugates may be provided as part of a test kit with one or more containers such as vials or bottles. Each container or vial contains a separate reagent such as a diluent, indicator reagent comprising a signal generating compound, assay reagents comprising a hapten-peptide conjugate as taught herein, and the like. The hapten-peptide conjugate reagent would be a substantially pure form of SEQUENCE I.D. NOS. 1-5 labeled with at least one N-terminal hapten. Such a test kit would also include instructions which indicate that the contents thereof may be used to detect/confirm the presence of the analyte of interest.

The peptides which are haptenated as taught herein are derived from the immunodominant region (IDR) of an HIV antigen such as HTV-1 gp41 IDR and HIV-2 gp36 IDR. These HIV peptides include:
SEQUENCE LD. NO.1: Lys-Asp-Gln-Gln-Leu-Leu-Gly-Ile-Trp-Gly-Cys-Ser-Gly-Lys-Leu-Ile-Cys-Thr-Thr, which is a 19-mer derived from the immunodominant region (IDR) of HIV-1 gp41 subtype B;
SEQUENCE LD. NO. 2: Tyr-Leu-Lys-Asp-Gln-Ala-Gln-Leu-Asn-Ser-Trp-Gly-Cys-Ala-Phe-Arg-Gln-Val-Cys-His-Thr-Thr-Val-Pro-Trp, which is a 25-mer derived from HIV-2 gp 36;
SEQUENCE LD. NO. 3: Arg-Ile-Leu-Ala-Val-Glu-Arg-Tyr-Leu-Lys-Asp-Gln-Gln-Leu-Leu-Gly-Ile-Trp-Gly-Cys-Ser-Gly-Lys-Leu-Ile-Cys-Thr-Thr which is a 28-mer derived from HIV-1 gp41; and
SEQUENCE I.D. NO. 4: Lys-Gln-Asp-Gln-Gln-Leu-Leu-Ser-Ile-Trp-Gly-Cys-Lys-Gly-Lys-Leu-Ile-Cys-Tyr-Thr, which is a 20-mer derived from the IDR of HIV-1 gp41, Type 0.
SEQUENCE LD. NO. 5: Lys-Asp-Gln-Gln-Leu-Leu-Gly-Ile-Trp-Gly-Cys-Lys-Gly-Lys-Leu-Ile-Cys-Tyr-Thr which is a 19-mer having a disulphide bridge between the cysteine at position 11 and the cysteine at position 17. The peptide is derived from the IDR of HIV-1 gp41, subtype B with a Ser to Lys change at position 12 and Thr to Tyr change at position 18.

Biotinylated forms of these peptides (antigens) are:
SEQUENCE LD. NO. 6: Xaa-Asp-Gln-Gln-Leu-Leu-Gly-Ile-Trp-Gly-Cys-Ser-Gly-Lys-Leu-Ile-Cys-Thr-Thr, wherein Xaa is N-e-(biotin-amidocaproyl)lysine;
SEQUENCE I.D. NO. 7: Xaa-Asp-Gln-Gln-Leu-Leu-Gly-De-Trp-Gly-Cys-Ser-Gly-Lys-Leu-Ile-Cys-Thr-Thr, wherein Xaa is N-α-(biotin-amidocaproyl)lysine;
SEQUENCE LD. NO. 8: Xaa-Tyr-Leu-Lys-Asp-Gln-Ala-Gln-Leu-Asn-Ser-Trp-Gly-Cys-Ala-Phe-Arg-Gln-Val-Cys-His-Thr-Thr-Val-Pro-Trp, wherein Xaa is N-ε-(biotin-amidocaproyl)lysine;
SEQUENCE I.D. NO. 9: Xaa-Tyr-Leu-Lys-Asp-Gln-Ala-Gln-Leu-Asn-Ser-Trp-Gly-Cys-Ala-Phe-Arg-Gln-Val-Cys-His-Thr-Thr-Val-Pro-Trp, wherein Xaa is N-α-(biotin-amidocaproyl)lysine;
SEQUENCE I.D. NO. 10: Xaa-Ile-Leu-Ala-Val-Glu-Arg-Tyr-Lzu-Lys-Asp-Gln-Gln-Leu-Leu-Gly-Ile-Trp-Gly-Cys-Ser-Gly-Lys-Leu-Ile-Cys-Thr-Thr, wherein Xaa is N-α-(biotin-amidocaproyl)arginine; and
SEQUENCE I.D. NO. 11: Xaa-Gln-Asp-Gln-Gln-Leu-Leu-Ser-Ile-Trp-Gly-Cys-Lys-Gly-Lys-Leu-Ile-Cys-Tyr-Thr, wherein Xaa is N-α-(biotinamidocaproyl)-lysine.
SEQUENCE LD. NO. 12: Xaa-Asp-Gln-Gln-Leu-Leu-Gly-De-Trp-Gly-Cys-Lys-Gly-Lys-Leu-Ile-Cys-Tyr-Thr wherein Xaa is N-α-(biotinamidocaproyl)-lysine.
SEQUENCE LD. NO. 13: Xaa-Asp-Gln-Gln-Leu-Leu-Gly-Ile-Trp-Gly-Cys-Lys-Gly-Lys-Leu-Ile-Cys-Tyr-Thr wherein Xaa is N-ε-(biotinamidocaproyl)-lysine.
SEQUENCE LD. NO. 14: Xaa-Asp-Gln-Gln-Leu-Leu-Gly-Ile-Trp-Gly-Cys-Lys-Gly-Lys-Leu-Ile-Cys-Tyr-Thr wherein Xaa is N-e-(biotinamidocaproyl)-α-(biotinamidocaproyl) lysine.

Peptide-conjugates employed according to the improved assay may be synthesized using solid phase peptide synthesis (SPPS). Using this process, biotin, for example, can be incorporated controllably and easily at a selected or pre-defined location (amino acid) within the peptide. SPPS can take advantage of chemical differences between acid labile and base labile protecting groups to site specifically incorporate haptens into amino acid residues. The biotinylation process disclosed herein is particularly useful in tagging peptide or protein antigens, which contain specific binding domains (epitopes) for antibodies. For such peptides, it is important that the biotin be incorporated at a location outside of the epitope region such that it will not interfere with antigen-antibody interaction.

In accordance with standard SPPS procedures, a peptide is synthesized on a solid support such as a resin. Suitable resins for supporting SPPS are well-known in the art and include , but are not limited to p-alkoxybenzyl alcohol resin and chloromethylated polystyrene resin. Peptide synthesis begins with the attachment of a desired carboxyl-terminal (C-terminal) amino acid to the resin. The peptide is synthesized by the sequential addition of amino acid residues to the C-terminal residue attached to the resin. Added amino acids form α-peptide links with the resin-supported residues. Other means for adding additional residues are well known in the art and include the Merrifield tBoc SPPS procedure. In a preferred embodiment, the peptide is built using repeated cycles of base-labile-N-α-Fmoc-amino acid residues. Thus, the C-terminal residue and all added residues, but not including the N-terminal residue, have a base-labile protecting group at their α-amino group.

All reactive groups of added residues are protected with a suitable protecting group in order to prevent undesirable conjugations between the added residues and the residue already present in the resin-bound chain. Suitable protecting groups for reactive groups of amino acids, including base-labile and acid-labile groups, are well known in the art (See, e.g., Protective Groups in Organic Synthesis, 2d Ed., T.W. Greene and P.G.M. Wuts, John Wiley & Sons, Inc., 1991). For example, the reactive sulfur (S) of cysteine, the β-amino group of asparagine, the γ-amino group of glutamine, and the imidazole nitrogen (N) of histidine can be protected with triphenylmethyl, known in the art as trityl (Trt). The reactive oxygen (O) of serine or threonine, the reactive O of tyrosine, the β-carboxyl group of aspartic acid, and the γ-carboxyl group of glutamic acid can be protected with t-butyl esters (tBu).

Means for selecting a suitable protecting agent are known in the art and depend on the method used to build the peptide chain and the amino acids comprising the peptide. Where a peptide chain is built using multiple base-labile α-protected amino acid residues, suitable protecting groups for other (non-α-amino) groups should be stable under those basic conditions used to add amino acid residues. This is particularly true of the process disclosed herein where the biotinylation process takes advantage of the differences in stability and chemical reactivity between protecting groups.

Additional amino acid residues, except the N-terminal amino acid residue, are added to the resin-bound peptide in a preselected order, depending on the desired sequence of the peptide to biotinylated, using the above procedure. A biotinylatable N-terminal amino acid residue is added to the resin-bound peptide chain. As used herein, the term "biotinylatable" means capable of being biotinylated. Amino acid residues capable of being biotinylated are well known in the art; a preferred amino acid residue is lysine.

The N-terminal amino acid typically is protected in such a manner that the desired site of biotinylation is protected with a base-labile protecting group and all other reactive groups on that residue are protected with an acid-labile protecting group. Thus, when the N-terminal residue is added to the resin-bound peptide chain using the multiple base-labile cycling procedure set forth above, the biotinylation site is unprotected while all other groups are blocked from reacting with the biotinylation step to follow. Base-labile protecting groups may include a carbamate such as 9-fluorenylinethoxycarbonyl (Fmoc), and acid-labile protecting groups may include trityl (Trt) and t-butoxycarbonyl (tBoc). It will be understood that the protecting groups employed are not intended to limit the present invention.

At the end of the peptide building steps set forth hereinabove, peptide of a predetermined amino acid residue sequence and a biotinylatable N-terminal residue exists bound to a resin and all reactive groups of the individual amino acid residues in that sequence are protected with an acid-labile protecting group. The reactive group of the N-terminal amino acid residue that is to be biotinylated is unprotected. The resin-bound, protected peptide is biotinylated by exposing that peptide to a biotinylation solution. Biotinylation solutions suitable for biotinylating peptides are known in the art; in a preferred embodiment, that solution comprises an N-hydroxysuccinimide ester of biotin or a biotinamidocaproate. Such esters can be obtained from various commercial sources such as Sigma Chemical Co., St. Louis, MO. In a preferred embodiment, an N-hydroxysuccinimide ester of a biotinamidocaproate of the formula biotin-(NH-CH₂-CH₂CH₂-CH₂-CH₂-CO)ₙ-ONHS is used, where n is 0 to 5 and preferably, n is 1 to 2. The biotinylation reaction is carried out in a suitable solvent as is known in the art. A preferred solvent is N-methylpyrrolidone (NMP).

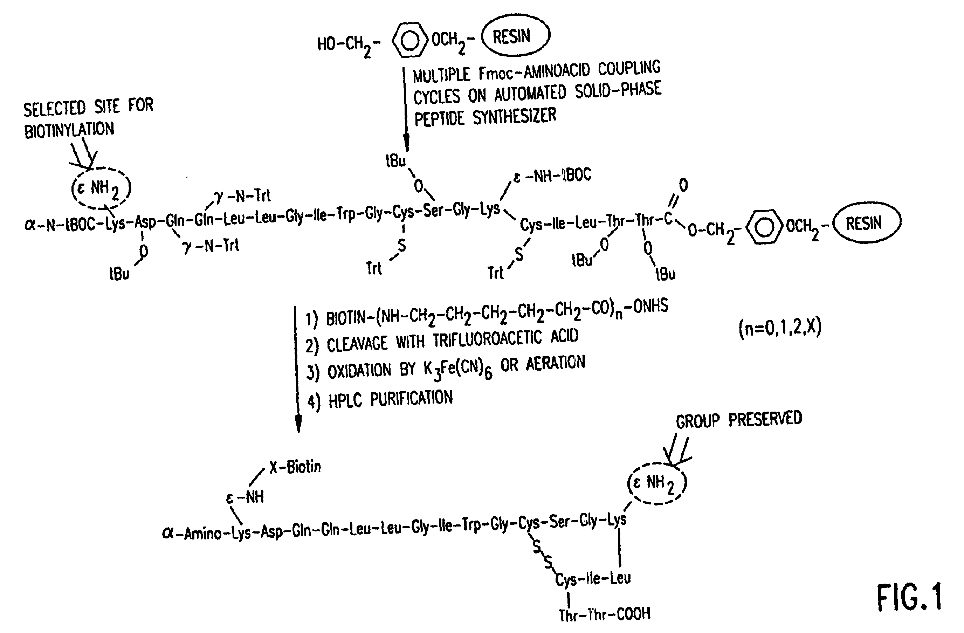

Following biotinylation of the peptide, the peptide is cleaved from the resin using standard procedures known in the art. In a preferred embodiment, cleaving is accomplished by treating the peptide with a cleaving solution containing trifluoroacetic acid (TFA). The cleaving solution can further comprise 1,2-ethanedithiol (EDT), anisole and dimethyl sulfide. The peptide is exposed to the cleaving solution for from about one to about five hours depending on the number of arginine residues in the peptide. The cleaved, biotinylated peptide then is recovered, preferably after oxidizing the peptide and purifying the biotinylated peptide. Oxidation of the cleaved peptide is accomplished typically by aerating an alkalinized solution of the peptide or by exposing the peptide to an oxidation agent such as K₃Fe(CN)₆ (potassium ferricyanide). Following oxidation, the peptide solution is acidified and purified using standard techniques. A preferred means of purification is by high pressure liquid chromatography (HPLC). A schematic diagram of the biotinylation process of the present invention to make a biotinylated HIV antigen is shown in FIGURE 1. A detailed description of the biotinylation of numerous peptides using a biotinylation process of the present invention is set forth hereinafter in the following examples.

The following examples illustrate embodiments of the present invention and are not meant to be limiting of the claims and specification in any way.

### EXAMPLES

### Example 1. Solid Phase Synthesis of SEQUENCE I.D. NO. 6

Synthesis of peptide SEQUENCE LD. NO. 6 was carried out on an automated solid phase peptide synthesizer using standard scale Fmoc chemistry with 0.25 mmol p-hydroxymethylphenoxymethyl (HMP) resin and 1.00 mmol Fmoc amino acids. The run editor was modified in that double coupling was introduced for each after ten amino acids attached. The added lysine residue located between the two cysteine residues was N-α-Fmoc-N-ε-Boc-L-Lysine, whereas the lysine residue at the N-terminal was N-α-Boc-N-ε-Fmoc-L-Lysine. The ε-amine of the N-terminal lysine was derivatized with biotinamidocaproate N-hydroxysuccinimide ester while the peptide was still on the resin. No deprotection was done except for the terminal amine at this time.

Specifically, about 160 mg (0.05 mmol) of the completed peptide on the resin was mixed in about 5 ml of an N-methylpyrrolidone (NMP) solvent system. About 45 mg (0.1 mmol) of biotinamidocaproate N-hydroxysuccinimide ester was added and the solution was mixed for about 6 hours. The peptide on the resin then was filter washed with about 2 ml methylene chloride and dried *in vacuo*. The peptide was cleaved with about 10 ml of a cleaving solution (10 ml) for about 120 minutes and filtered. The cleaving solution was a mixture of 95% trifluoroacetic acid (TPA) and 5% of a 1:3:3 mix of 1,2-ethanedithiol (EDT):Anisole:dimethyl sulfide. The peptide was filtered from the resin and precipitated with cold ether. The precipitated peptide was filtered and washed with ether. The crude peptide was dissolved in dimethylformamide (DMF)-water, the pH was adjusted to about 9.0 with sodium carbonate and a steady stream of air was passed through the DMF solution (i.e., the peptide was oxidized).

Following oxidation, the solution was acidified with TFA and the biotinylated peptide purified using high pressure liquid chromatography (HPLC). The peaks were separated on a C18-Reverse Phase-HPLC column, 25.4 mm x 25 cm, 300A, using a 20% to 100% gradient of acetonitrile-water with a 0.1% trifluoroacetic acid (TFA) solvent system. The flow rate was 12 ml/min. Ultraviolet (UV) detection utilized 230 nm and 260 nm wavelengths. The isolated material (11 mg) was analyzed by mass spectometry (MS) and showed a molecular ion at MS 2404 for the correct product. The biotinylated peptide was analyzed for free thiol content by Ellmans reagent (available from Sigma Chemical Co.; St. Louis MO). Only 7% of free thiol was present in the purified peptide.

### Example 2. Solid-Phase Synthesis of SEQUENCE I.D. NO. 7

Biotinylated peptide SEQUENCE I.D. NO. 7 was prepared as described above in Example 1 with the exception that both lysine residues (the lysine located between the cysteine residues and the N-terminal lysine) were added to the resin-bound chain as N-ε-tBOC-N-α-Frnoc-Lys. The biotin-amidocaproic acid was coupled to the α-amino group of the N-terminal lysine.

### Example 3. Solid Phase Synthesis of SEOUENCE I.D. NO. 8

Biotinylated peptide SEQUENCE LD. NO. 8 was prepared as described above in Example 1.

### Example 4. Solid-phase Synthesis of SEQUENCE I.D. NO. 9

Biotinylated peptide SEQUENCE I.D. NO. 9 was prepared as described above in Example 1.

### Example 5. Solid-phase Synthesis of SEQUENCE I.D. NO. 10

Biotinylated peptide SEQUENCE I.D. NO. 10 was prepared according to Example 1 except that both Lys residues were incorporated as N-α-tBOC-N-ε-Fmoc-Lys. The N-terminal Arg residue was added as N-ε-Fmoc-N-α-(2,2,5,7,8-pentamethylchroman-6-sulfonyl)-L-arginine. The biotin was coupled to the α-amino group of the N-terminal Arg. SEQUENCE LD. NO. 10 was purified from HPLC with a mass spectrum molecular Wt. of 3405.

### Example 6. Solid-Phase Synthesis of SEQUENCE I.D. NO. 11

Biotinylated peptide SEQUENCE I.D. NO. 11 was prepared according to Example 1 except that both Lys residues were incorporated as N-ε-tBOC-N-α-Fmoc-Lys. The biotin-amidocaproic acid was coupled to the α-amino group of the N-terminal lysine.

### Example 7. Chemiluminescent Immunoassay for HIV-1 Antibodies

Biotinylated peptide SEQUENCE LD. NO. 6 was prepared as described in Example 1. A solution biotinylated peptide SEQUENCE LD. NO. 1 was made by dissolving about 1 mg of SEQUENCE LD. NO. 1 (Custom synthesized by Cambridge Research Biochemicals, Cheshire, England) in about 1 ml of a sodium bicarbonate solution (20 mM, pH 8.0). The peptide solution was mixed with an N-hydroxylsuccinimide ester of biotin-amidocaproyl-amidocaproic acid (1.23 mg, 4.5 molar equivalents to the 19-mer) by magnetic stirring at ambient temperature overnight.

Although the biotinylated peptide can be further processed to remove excess biotin by dialysis in tubing with a low molecular weight cut-off of 1,000, we determined that the biotinylated peptide can be used without removal of free biotin in an immunoassay system with a wash step and when using a monoclonal anti-biotin antibody, which has a much higher binding affinity to macromolecule-linked-biotin than to free biotin.

The assay was run on a stand-alone, semi-automated instrument (O.S. Khalil et al., Clin, Chem 37, pp. 1540-1547,1991) (Abbott PRISM™ instrument, available from Abbott Laboratories, Abbott Park, IL 60064). The instrument system was modified to a high throughput version of 40 second cycles per step instead of 72 second cycles as described in the cited reference.

An HIV antibody assay was performed as follows. First, 50 µl of a blend of microparticles coated with rDNA antigens for HIV-1 and HIV-2 was incubated with 100 µl of test sample in the incubation well to form a mixture for 18 minutes. Each type of microparticle in the blend were separately coated with the rDNA antigens by passive adsorption on polystyrene latex microparticles available from Seradyn; Indianapolis IN. After coating, the blended particles were heat stressed at 55°C-60°C. Next, the entire sample/microparticle mixture was transferred with 600 µl of transfer buffer (pH 7.2, 10 mM phosphate, 0.9% NaCl, 0.12% non-fat dry milk and 0.1 % NaN₃) from the incubation well to the detection well. Then, biotinylated peptide SEQUENCE I.D. NO. 6 or biotinylated peptide SEQUENCE I.D. NO.1 (prepared as described above in Example 1 and 7, respectively) was dissolved at a concentration of about 3.3 µ/ml, in pH 8.3, 0.1 M borate, 6% calf serum, 1% *E. coli* lysate, 2% cholic acid, 0.05% CKS (CTP:CMP-3-deoxy-manno-octulosonate cytidylyl transferase or CMP-KDO synthetase), and 0.1% NaN₃. About 50 µl of the biotinylated peptide solution was pipetted into the detection well. Following this, the reaction mixture was incubated for about 10.6 minutes. Each sample well then was washed four times with about 100 µl of a probe wash buffer (pH 9.0, 0.1 M borate, 0.25 M NaCl, 0.025% lithium lauryl sulfate, and 0.1% NaN₃). Then, about 50 µl of an indicator reagent conjugate (N-methyl acridinium conjugate of monoclonal anti-biotin (120 ng/ml in pH 6.3 phosphate buffered saline [PBS], 4% bovine serum albumin [BSA], 1% Triton X-100®, 0.2% non-fat dry milk, and 0.1% NaN3) was added to each reaction well. Next, the reaction mixtures were incubated for about 10.6 minutes. Following this incubation, each well was washed with a conjugate buffer of pH 5.7 containing 25 mM (2[N-morpholino]ethanesulfonic acid) (MES), 0.9% NaCl, and 0.1% Proclin®). Samples then were incubated for about 5 minutes while being moved to the station for chemical triggering and photon detection. The reaction mixture in the reaction well was triggered with about 50 µl of urea peroxide (0.2% in 0.15 N NaOH), and photon counts were integrated with a photo-multiplier. The results of this study are shown in TABLE 1.

As the data from TABLE 1 demonstrate, the use of SEQUENCE LD. NO.6 resulted in a significant increase in the signal to noise ratio (S/N) when compared to results using the solution biotinylated peptide SEQUENCE I.D. NO.1.

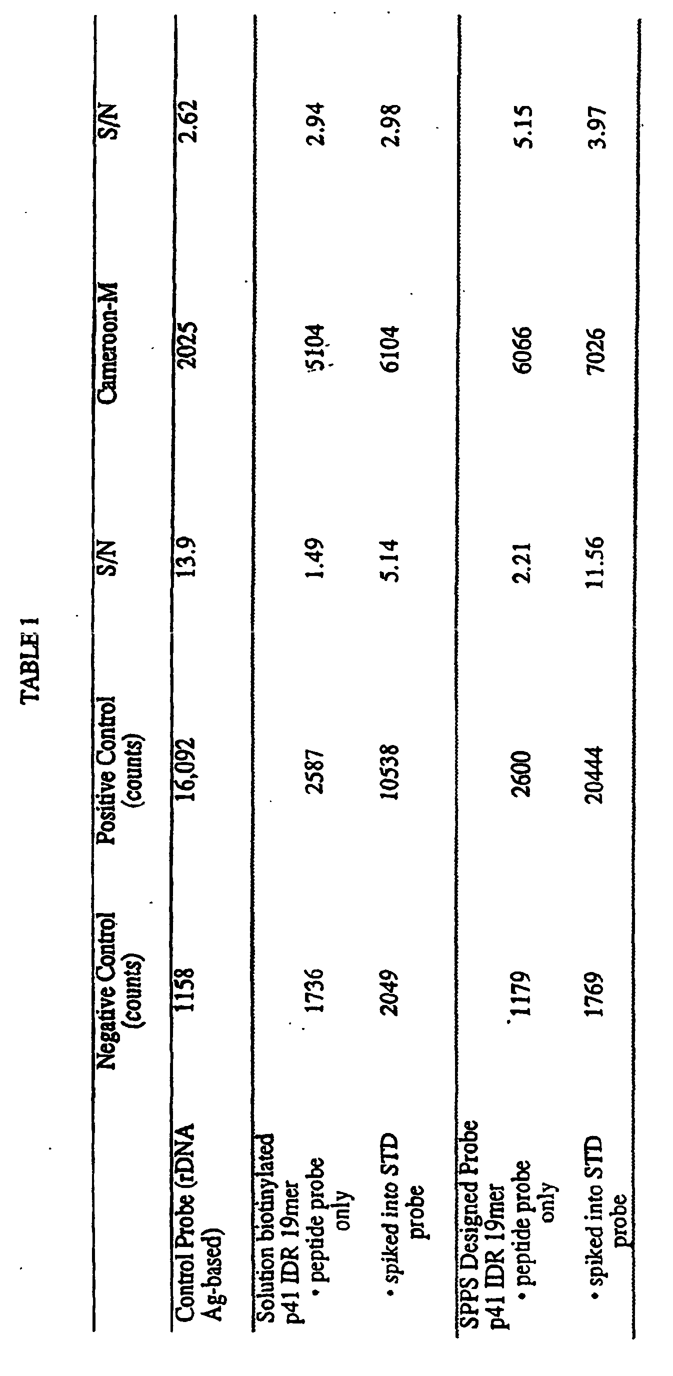

### Example 8. Dose Responses

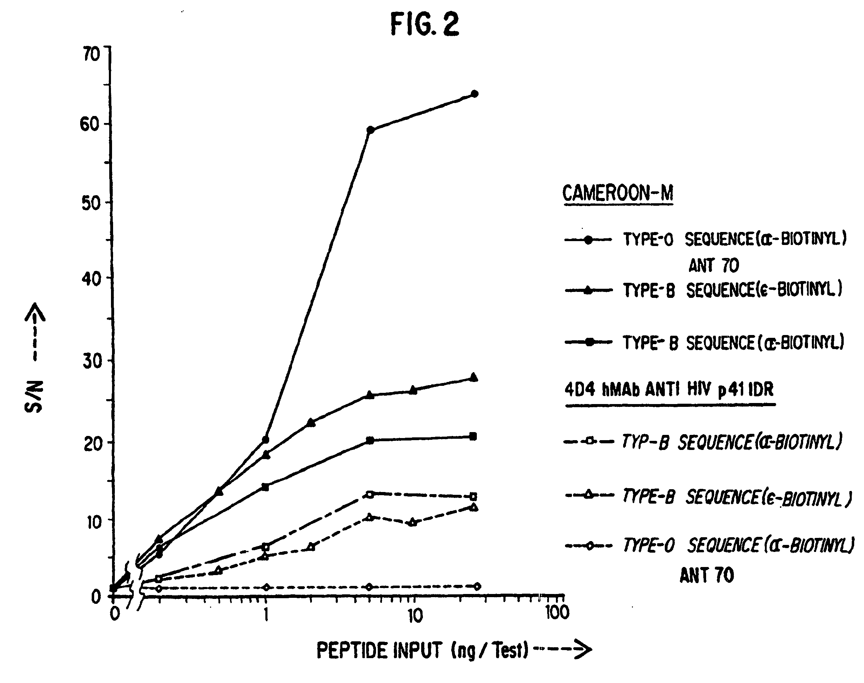

A. Subtype O HIV-1 test sample responses to biotinylated HIV-1 gp41 IDR peptides in a gp41 peptide-only probe assay. A chemiluminescent inununoassay was performed following the procedures of Example 7, except that step 3 of the assay used a series of dilutions: (i) SEQUENCE I.D. NO. 6 (ε-N-terminal biotinylated HIV-1 type B sequence) in concentrations of 0, 4, 10, 20, 40, 100, 200 and 500 ng/ml, (ii) SEQUENCE LD. NO. 7 (α-N-terminal biotinylated HIV-1 type B sequence) in concentrations of 4, 20, 100, and 500 ng/ml, and (iii) SEQUENCE I.D. NO. 11 (α-N-terminal biotinylated HIV-1 type O sequence) in concentrations of 4, 20, 100 and 500 ng/ml. Controls and samples used in the run were negative calibrator, a gp41 IDR panel (monoclonal IAM 41-4D4; A. Buchacher, et al., AIDS Res Hum Retroviruses, 10:359-369, [1994]) and HIV-1 Subtype-O panel (Cameroon-M). The results of this experiment are presented in FIGURE 2. As can be seen in FIGURE 2, the data show the sensitivity of an immunoassay using a biotinylated peptide of the present invention.

### B. Dose Responses of anti-HIV-1 Samples to SEQUENCE I.D. NO. 6 in a FHV-1/2 Combined Whole Assay.

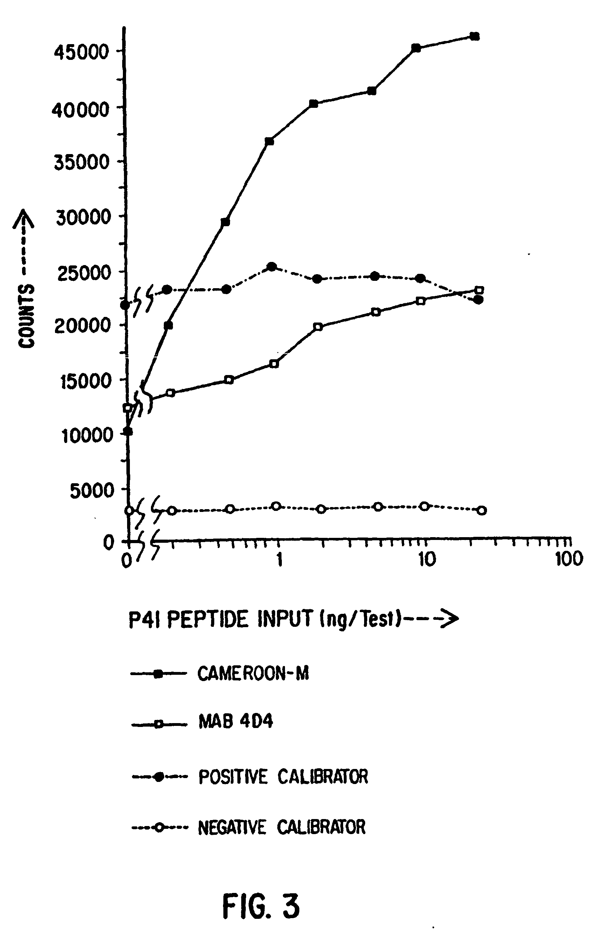

The assay protocol as described in Example 7 was followed except that the probe at step 3 was using a series of eight different probes made from a base probe consisting of (a) 3 biotinylated Arg of HIV-1 gp41, HIV-1 p24, and HIV-2,(2.8, 0.6, and 0.2 µg/ml, respectively) and (b) biotinylated 19-mer of HIV-2 IDR (0.05 µg/ml); then, spiked with the biotinylated HIV-1 gp41 IDR 19-mer to concentrations of 0, 4, 10, 20, 40, 100, 200, and 500 ng/ml. Controls and test samples used in the run were negative calibrator, positive calibrator, gp41 IDR panel (MAb 4D4, *supra),* and HIV-1 Subtype-O Panel (Cameroon-M). A dose response curve of test samples to SEQUENCE LD. NO. 6 is shown in FIGURE 3, in a semi-log plot of counts versus input of peptide. The data from FIGURE 3 show the sensitivity of an immunoassay using a biotinylated peptide of the present invention.

### Example 9. Solid Phase Synthesis of SEQUENCE I.D. NO. 12

The fully protected peptide of SEQUENCE I.D. NO. 5 was assembled on a hydroxymethylphenyl (HMP) resin by stepwise solid phase synthesis essentially as described in Example 1. The following protected amino acids were used in the synthesis:
Fmoc-(tBu)-Asp-OH
Fmoc-(Trt)-Cys-OH
Fmoc-(Trt)-Gln-OH
Fmoc-Gly-OH
Fmoc-Ile-OH
Fmoc-Leu-OH
Fmoc-(tBoc)-Lys-OH
Fmoc-(tBu)-Thr-OH
Fmoc-Trp-OH

All of the amino acids were double-coupled at a 4-fold molar excess over reactive sites.

Biotinylation of the alpha amino position of the N-terminal lysine proceeded by first removing the terminal Fmoc protecting group with 20% piperidine in NMP. Biotin was then coupled to the alpha amino terminus by the addition of a 1 mole equivalent of biotinamidocaproyl-N-hydroxysuccinimide ester to the fully protected peptide while still on the resin. The peptide-resin complex was washed with NMP and the coupling repeated. Coupling was performed while vortexing the reagents on the automated peptide synthesizer in 10mls of NMP for 24 hours.

The peptide was then cleaved from the resin as in Example 1 and the remaining protecting groups removed by stirring the protected peptide with 92.5% trifluoroacetic acid, 5% ethanedithiol, 2.5% water for 6 hours at room temperature. The peptide was then filtered from the resin and precipitated with cold ether. The precipitated peptide was filtered and washed with ether.

The crude peptide was analyzed for purity using reversed-phase high performance liquid chromatography on a C18, 4.6mm x 25cm column using a flow rate one ml/min employing 0.1% aqueous TFA as "solvent A" and 0.1% TFA in aretonitrile as "solvent B". The solvent gradient employed for this peptide analysis started with 20% solvent B. A linear gradient of 1%/min to 70% solvent B was used to elute the peptide followed by 10 minutes of 100% solvent B to wash the column. The presence of peptide in the effluent was monitored simultaneously at 230nm and 260nm.

Preparative scale reversed phase high performance liquid chromatography was performed in a similar manner using a C18, 41.4mm x 25cm column, using the solvent system described above. The peptide fractions were collected as they eluted and lyophilised to dryness before mass spectral data were collected.

The intramolecular disulfide bond between the lysine located at position 12 and the tyrosine located at position 18 was formed by stirring the purified peptide in 20% dimethylsulfoxide, 80% 100mM tris® buffer pH 6.0 at a concentration of Img/ml or less. Analytical reversed phase high performance liquid chromatography was used to monitor the extent of reaction. When oxidation was complete (approximately 24 hours) the peptide was purified by preparative scale reversed phase high performance liquid chromatography as described above. Mass spectral data of the finished product confirmed the correct product with a molecular weight of 2504.9.

### Example 10. Solid Phase Synthesis of SEQUENCE I.D. NO. 13

The synthesis of SEQUENCE LD. NO. 13 proceeded in essentially the same manner as described in Example 9. However, the N-α-(FMOC)-N-ε-(biotin-amidocaproyl)-Lys replaced N-α-(FMOC)-N-ε-(tBoc)-Lys at the N-terminal position of the sequence. The biotinylated lysine derivative was double-coupled at 1 mole equivalent of reactive sites. Additionally, the alpha amino terminus was not biotinylated in this peptide.

The remaining steps; cleavage, purification of crude product by reversed phase high performance liquid chromatography, oxidation, and repurification by HPLC, were the same.

### Example 11. Solid Phase Synthesis of SEOLIENCE I.D. NO. 14

The synthesis of SEQUENCE LD. NO. 14 proceeded in essentially the same manner as described in Example 9. However, N-α-(FMOC)-N-ε-(FMOC)-Lys replaced N-α-(FMOC)-N-ε-(tBoc)-Lys at the N-terminal position of the sequence. Additionally, biotinylation at the alpha and epsilon positions of the N-terminal lysine occurred simultaneously with the addition of 2 mole equivalents of biotinamidocaproyl-N-hydroxysuccinimide to the peptide as outlined above. This coupling was repeated with another 2 mole equivalents of biotin reagent.

The remaining steps; cleavage, purification of crude product by reversed phase high performance liquid chromatography, oxidation, and repurification by HPLC, were the same.

### Example 12. MEIA for HIV-1 and HIV-2 Antibodies Using Site Specific and Quasi Solution Biotinylated Peptide Conjugates

The site specific biotinylated peptides synthesized in Examples 9-11 were compared to peptides representative of those obtained by solution phase biotinylation. SEQUENCE I.D. NO. 15, SEQUENCE LD. NO. 16 and SEQUENCE LD. NO. 17 were synthesized using standard FMOC chemistry using biotinylated lysine residues for labeling the peptide at postions 12, 14 or 12 and 14.

A microparticle enzyme immunoassay for FHV-1 and HIV-2, was used to compare the site specifically biotinylated peptides to those representative of solution biotinylated peptides. The assays were run on an AxSYM® analyzer which is an automated system commercially available from Abbott Laboratories (Abbott Park, IL). The AxSYM® analyzer combined human serum samples with microparticles coated with HIV-1 and HIV-2 recombinant proteins (capture reagent). Thus, anti-HIV-1 and anti-HIV-2 antibodies present in the test sample bound to the solid phase. The capture reagent/antibody complexes were then contacted and incubated with the solution biotinylated peptide conjugate or the site speafically biotinylated peptide conjugate to form capture reagent/antibody/peptide conjugate complexes. In order to detect any complexes, rabbit anti-biotin antibody conjugated to alkaline phosphatase was then incubated with the capture/antibody/peptide conjugate complexes. After a wash, 4-methylumbelliferyl-phosphate was added. The rate of appearance of a fluorescent dephosphorylated substrate correlated with the detection of HIV antibodies. The capture reagent, alkaline phosphatase conjugate, and 4-methylumbelliferyl-phosphate substrate are commercially available from Abbott Laboratories. Data for the various assays is shown in Table 2 below. As shown by Table 2, the site specifically biotinylated peptide conjugates (SEQUENCE LD. NO. 12, SEQUENCE LD. NO. 13 and SEQUENCE LD. NO. 14) gave superior results as compared to peptide conjugates representative of those obtained through solution biotinylation of a similar peptide having an amino acid residue distinction at position 18 (SEQUENCE I.D. NO. 15, SEQUENCE I.D. NO. 16 and SEQUENCE LD. NO. 17).

**Table 2 Rates**

| Sample # | Seq. LD. No. 12 | Seq. I.D. No. 13 | Seq. I.D. No. 14 | Seq. I.D. No. 15 | Seq. LD. No. 16 | Seq. LD. No. 17 |
|---|---|---|---|---|---|---|
| 1 | 42.21 | 42.11 | 88.57 | 21.53 | 22.45 | 18.12 |
| 2 | 35.76 | 38.10 | 71.10 | 14.83 | 12.40 | 11.73 |
| 3 | 12.20 | 16.36 | 21.88 | 13.63 | 12.37 | 14.55 |
| 4 | 148.29 | 177.72 | 94.63 | 15.51 | 11.47 | 10.98 |

As the data hereinabove demonstrate, biotinylated HIV peptides disclosed and prepared as described hereinabove gave superior results in detecting anti-HIV antibodies when compared to the use of a biotinylated peptide prepared using the solution biotinylation procedure.

While the invention has been described in detail and with reference to specific embodiments, it will be apparent to one skilled in the art that various changes and modifications may be made to such embodiments without departing from the scope of the invention.

## Claims

1. An immunoassay designed to detect the presence of anti-HIV-1 or anti-HIV-2 antibody in a test sample, said assay comprising the steps of:
(i) contacting said test sample with a capture reagent to form capture reagent / anti-HIV-1 or anti-HIV-2 antibody complexes;
(ii) contacting said capture reagent / anti-HIV-1 or anti-HIV-2 antibody complexes with at least one hapten-peptide conjugate to form capture reagent / anti-HIV-1 or anti-HIV-2 antibody / hapten-peptide conjugate complexes;
(iii) contacting said capture reagent / anti-HIV-1 or anti-HIV-2 antibody / hapten-peptide conjugate complexes with an indicator reagent to form capture reagent / anti-HIV-1 or anti-HIV-2 antibody / hapten-peptide conjugate / indicator reagent complexes; and
(iv) detecting a signal generated by said indicator reagent as an indication of the presence of said anti-HIV-1 or anti-HIV-2 antibody in said test sample;
**characterized in that** said peptide in said hapten-peptide conjugate is selected from the group consisting of the following sequences:
and said hapten is at least one hapten located at the N-terminal α position and/or ε position.

2. The immunoassay of claim 1 wherein said hapten comprises biotin.

3. A composition useful for detecting anti-HIV-1 or anti-HIV-2 antibodies wherein said composition comprises a substantially pure haptenated peptide sequence **characterized in that** said peptide is selected from the group consisting of the following peptides:
and said hapten is at least one hapten located at the N-terminal α position and/or ε position.

4. The composition of claim 3 wherein said hapten comprises biotin.

5. A test kit useful for determining the presence of an anti-HTV-1 or an anti-HIV-2 antibody analyte in a test sample, said kit comprising a container containing a substantially pure haptenated peptide sequence **characterized in that** said peptide is selected from the group consisting of the following peptides:
and said hapten is at least one hapten located at the N-terminal a position and/or ε position.

6. The kit of claim 5 wherein said hapten comprises biotin.

## Patentansprüche

1. Ein Immunoassay, der ausgelegt ist, um die Anwesenheit von Anti-HIV-1- oder Anti-HIV-2-Antikörper in einer Testprobe nachzuweisen, wobei der Assay die folgenden Schritte umfasst:
(i) In-Kontakt-bringen der Testprobe mit einem Einfangreagenz, um Einfangreagenz/Anti-HIV-1- oder Anti-HIV-2-Antikörper-Komplexe zu bilden;
(ii) In-Kontakt-bringen der Einfangreagenz/Anti-HIV-1- oder Anti-HIV-2-Antikörper-Komplexe mit mindestens einem Hapten-Peptid-Konjugat, um Einfangreagenz/Anti-HIV-1- oder Anti-HIV-2-Antikörper/Hapten-Peptid-Konjugat-Komplexe zu bilden;
(iii) In-Kontakt-bringen der Einfangreagenz/Anti-HIV-1- oder Anti-HIV-2-Antikörper/Hapten-Peptid-Konjugat-Komplexe mit einem Indikatorreagenz, um Einfangreagenz/Anti-HIV-1- oder Anti-HIV-2-Antikörper/Hapten-Peptid-Konjugat/Indikatorreagenz-Komplexe zu bilden; und
(iv) Nachweisen eines Signals, das durch das Indikatorreagenz generiert wird, als eine Anzeige der Anwesenheit des Anti-HIV-1-oder Anti-HIV-2-Antikörpers in der Testprobe;
**dadurch gekennzeichnet, dass** das Peptid in dem Hapten-Peptid-Konjugat gewählt ist aus der Gruppe bestehend aus den folgenden Sequenzen: und das Hapten mindestens ein Hapten ist, das sich an der N-terminalen α-Position und/oder ε-Position befindet.

2. Der Immunoassay von Anspruch 1, worin das Hapten Biotin umfasst.

3. Eine Zusammensetzung, die nützlich ist zum Nachweisen von Anti-HIV-1- oder Anti-HIV-2-Antikörpern, worin die Zusammensetzung eine im wesentlichen reine haptenierte Peptidsequenz umfasst, die **dadurch gekennzeichnet ist, dass** das Peptid gewählt ist aus der Gruppe bestehend aus den folgenden Peptiden: und das Hapten mindestens ein Hapten ist, das sich an der N-terminalen α-Position und/oder ε-Position befindet.

4. Die Zusammensetzung von Anspruch 3, worin das Hapten Biotin umfasst.

5. Ein Testkit, der nützlich ist zum Bestimmen der Anwesenheit von Anti-HIV-1- oder Anti-HIV-2-Antikörper-Analyt in einer Testprobe, wobei der Kit einen Behälter umfasst, der eine im Wesentlichen reine haptenierte Peptidsequenz umfasst, die **dadurch gekennzeichnet ist, dass** das Peptid gewählt ist aus der Gruppe bestehend aus den folgenden Peptiden: und das Hapten mindestens ein Hapten ist, das sich an der N-terminalen α-Position und/oder ε-Position befindet. e

6. Der Kit von Anspruch 5, worin das Hapten Biotin umfasst.

## Revendications

1. Immunodosage conçu pour détecter la présence d'un anticorps anti-VIH-1 ou anti-VIH-2 dans un échantillon à tester, ledit dosage comprenant les étapes consistant à :
(i) mettre ledit échantillon à tester en contact avec un réactif de capture pour former des complexes réactif de capture / anticorps anti-VIH-1 ou anti-VIH-2 ;
(ii) mettre lesdits complexes réactif de capteur / anticorps anti-VIH-1 ou anti-VIH-2 en contact avec au moins un conjugué haptène-peptide pour former des complexes réactif de capture / anticorps anti-VIH-1 ou anti-VIH-2 / conjugué haptène-peptide ;
(iii) mettre lesdits complexes réactif de capture / anticorps anti-VIH-1 ou anti-VIH-2 / conjugué haptène-peptide en contact avec un réactif indicateur pour former des complexes réactif de capture / anticorps anti-VIH-1 ou anti-VIH-2 / conjugué haptène-peptide / réactif indicateur ; et
(iv) détecter un signal engendré par ledit réactif indicateur comme indication de la présence dudit anticorps anti-VIH-1 ou anti-VIH-2 dans ledit échantillon à tester ;
**caractérisé en ce que** ledit peptide dans ledit conjugué haptène-peptide est choisi dans le groupe constitué par les séquences suivantes :
et ledit haptène est au moins un haptène situé au niveau de la position α et/ou ε N-terminale.

2. Immunodosage selon la revendication 1, dans lequel ledit haptène comprend la biotine.

3. Composition permettant de détecter des anticorps anti-VIH-1 ou anti-VIH-2, laquelle composition comprend une séquence peptidique hapténée sensiblement pure
**caractérisée en ce que** ledit peptide est choisi dans le groupe constitué par les peptides suivants :
et ledit haptène est au moins un haptène situé au niveau de la position α et/ou ε N-terminale.

4. Composition selon la revendication 3, dans laquelle ledit haptène comprend la biotine.

5. Trousse de test permettant de déterminer la présence d'un analyte de type anticorps anti-VIH-1 ou anti-VIH-2 dans un échantillon à tester, ladite trousse comprenant un récipient contenant une séquence peptidique hapténée sensiblement pure **caractérisée en ce que** ledit peptide est choisi dans le groupe constitué par les peptides suivants :
et ledit haptène est au moins un haptène situé au niveau de la position α et/ou ε N-terminale.

6. Trousse selon la revendication 5, dans laquelle ledit haptène comprend la biotine.


### SEQUENCE LISTING

(1) GENERAL INFORMATION:
   (i) APPLICANT: David J. Daghfal
      Tracey L. Colpitts
      Barbara T. Merchant
      Chi D. Chang
      Isaac S. Y. Sze
      Keeve D. Jaffe
      Dominique Bridon
   (ii) TITLE OF INVENTION: IMPROVED HAPTEN-PEPTIDE CONJUGATES
   (iii) NUMBER OF SEQUENCES: 17
   (iv) CORRESPONDENCE ADDRESS:
      (A) ADDRESSEE: ABBOTT LABORATORIES D377/AP6D
      (B) STREET: 100 ABBOTT PARK ROAD
      (C) CITY: ABBOTT PARK
      (D) STATE: IL
      (E) COUNTRY: USA
      (F) ZIP: 60064-3500
   (v) COMPUTER READABLE FORM:
      (A) MEDIUM TYPE: Floppy disk
      (B) COMPUTER: Macintosh
      (C) OPERATING SYSTEM: System 7.0.1
      (D) SOFTWARE: Microsoft Word 5.1a
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: POREMBSKI, PRISCILLA E.
      (B) REGISTRATION NUMBER: 33,207
      (C) REFERENCE/DOCKET NUMBER: 5766.US.01
   (ix) TELECOMMUNICATION INFORMATION:
      (A) TELEPHONE: 708-937-6365
      (B) TELEFAX: 708-938-2623
(2) INFORMATION FOR SEQ ID NO:1:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Lys Asp Gln Gln Leu Leu Gly Ile Trp Gly Cys Ser Gly Lys Leu Ile
   1               5                   10                  15
   Cys Thr Thr
(2) INFORMATION FOR SEQ ID NO:2:
   (i) SEQUENCE CHARACTERISTICS:
      (A) LENGTH: 26 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Lys Tyr Leu Lys Asp Gln Ala Gln Leu Asn Ser Trp Gly Cys Ala Phe
   1               5                   10                  15
   Arg Gln Val Cys His Thr Thr Val Pro Trp
               20                  25
(2) INFORMATION FOR SEQ ID NO:3:
   (i) SEQUENCE CHARACTERISTICS:
      (A) LENGTH: 28 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Arg Ile Leu Ala Val Glu Arg Tyr Leu Lys Asp Gln Gln Leu Leu Gly
   1 5 10 15
   Ile Trp Gly Cys Ser Gly Lys Leu Ile Cys Thr Thr
   20 25
(2) INFORMATION FOR SEQ ID NO:4:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Lys Gln Asp Gln Gln Leu Leu Ser Ile Trp Gly Cys Lys Gly Lys Leu
   1 5 10 15
   Ile Cys Tyr Thr
   20
(2) INFORMATION FOR SEQ ID NO:5:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
   Lys Asp Gln Gln Leu Leu Gly Ile Trp Gly Cys Lys Gly Lys Leu Ile
   1 5 10 15
   Cys Tyr Thr
(2) INFORMATION FOR SEQ ID N0:6:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Epsilon-Biotin-Amidocaproyl-Lysine"
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa= N Alpha-Biotin-Amidocaproyl-Lysine"
   (xi) SEQUENCE DESCRIPTION: SEQ ID NO:7:
   Xaa Asp Gln Gln Leu Leu Gly Ile Trp Gly Cys Ser Gly Lys Leu Ile
   1               5                   10                  15
   Cys Thr Thr
(2) INFORMATION FOR SEQ ID NO:8:
   (i) SEQUENCE CHARACTERISTICS:
      (A) LENGTH: 26 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Epsilon-Biotin-Amidocaproyl-Lysine"
   (xi) SEQUENCE DESCRIPTION: SEQ ID NO:8:
   Xaa Tyr Leu Lys Asp Gln Ala Gln Leu Asn Ser Trp Gly Cys Ala Phe
   1               5                   10                  15
   Arg Gln Val Cys His Thr Thr Val Pro Trp
               20                  25
(2) INFORMATION FOR SEQ ID NO:9:
   (i) SEQUENCE CHARACTERISTICS:
      (A) LENGTH: 26 amino acids
      (B) TYPE: amino acid
      (C) STRANDEDNESS: single
      (D) TOPOLOGY: linear
   (ii) MOLECULE TYPE: peptide
   (x) FEATURE:
      (A) NAME/KEY: Modified-site
      (B) LOCATION: 1
      (D) OTHER INFORMATION: /label= Xaa
         /note= "Xaa = N Alpha-Biotin-Amidocaproyl-Lysine"
   (xi) SEQUENCE DESCRIPTION: SEQ ID NO:9:
   Xaa Tyr Leu Lys Asp Gln Ala Gln Leu Asn Ser Trp Gly Cys Ala Phe
   1               5                   10                  15
   Arg Gln Val Cys His Thr Thr Val Pro Trp
               20                  25
(2) INFORMATION FOR SEQ ID NO:10:
   (i) SEQUENCE CHARACTERISTICS:
      (A) LENGTH: 28 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Alpha-Biotin-Amidocaproyl-Arginine"
   (xi) SEQUENCE DESCRIPTION: SEQ ID NO:10:
   Xaa Ile Leu Ala Val Glu Arg Tyr Leu Lys Asp Gln Gln Leu Leu Gly
   1 5 10 15
   Ile Trp Gly Cys Ser Gly Lys Leu Ile Cys Thr Thr
   20 25
(2) INFORMATION FOR SEQ ID NO:11:
   (i) SEQUENCE CHARACTERISTICS:
      (A) LENGTH: 20 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Alpha-Biotin-Amidocaproyl-Lysine"
   (xi) SEQUENCE DESCRIPTION: SEQ ID NO:11:
   Xaa Gln Asp Gln Gln Leu Leu Ser Ile Trp Gly Cys Lys Gly Lys Leu
   1 5 10 15
   Ile Cys Tyr Thr
   20
(2) INFORMATION FOR SEQ ID NO:12:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Alpha-Biotin-Amidocaproyl-Lysine"
   (xi) SEQUENCE DESCRIPTION: SEQ ID NO:12:
   Xaa Asp Gln Gln Leu Leu Gly Ile Trp Gly Cys Lys Gly Lys Leu Ile
   1 5 10 15
   Cys Tyr Thr
(2) INFORMATION FOR SEQ ID NO:13:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Epsilon-Biotin-Amidocaproyl-Lysine"
   (xi) SEQUENCE DESCRIPTION: SEQ ID NO:13:
   Xaa Asp Gln Gln Leu Leu Gly Ile Trp Gly Cys Lys Gly Lys Leu Ile
   1               5                   10                  15
   Cys Tyr Thr
(2) INFORMATION FOR SEQ ID NO:14:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 1
      (D) OTHER INFORMATION: /label= Xaa
         /note= "Xaa = N Alpha-Epsilon-Biotin-Amidocaproyl-Lysine"
   (xi) SEQUENCE DESCRIPTION: SEQ ID NO:14:
   Xaa Asp Gln Gln Leu Leu Gly Ile Trp Gly Cys Lys Gly Lys Leu Ile
   1               5                   10                  15
   Cys Tyr Thr
(2) INFORMATION FOR SEQ ID NO:15:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 12
      (D) OTHER INFORMATION: /label= Xaa
         /note= "Xaa = Epsilon-Biotin-Amidocaproyl-Lysine"
   (xi) SEQUENCE DESCRIPTION: SEQ ID NO:15:
   Lys Asp Gln Gln Leu Leu Gly Ile Trp Gly Cys Xaa Gly Lys Leu Ile
   1               5                   10                  15
   Cys Thr Thr
(2) INFORMATION FOR SEQ ID NO:16:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 14
      (D) OTHER INFORMATION: /label= Xaa
         /note= "Xaa = Epsilon-Biotin-Amidocaproyl-Lysine"
   (xi) SEQUENCE DESCRIPTION: SEQ ID NO:16:
   Lys Asp Gln Gln Leu Leu Gly Ile Trp Gly Cys Lys Gly Xaa Leu Ile
   1               5                   10                  15
   Cys Thr Thr
(2) INFORMATION FOR SEQ ID NO:17:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 12
      (D) OTHER INFORMATION: /label= Xaa
         /note= "Xaa = Epsilon-Biotin-Amidocaproyl-Lysine"
   (ix) FEATURE:
      (A) NAME/KEY: Modified-site
      (B) LOCATION: 14
      (D) OTHER INFORMATION: /label= Xaa
         /note= "Xaa = Epsilon-Biotin-Amidocaproyl-Lysine"
   (xi) SEQUENCE DESCRIPTION: SEQ ID NO:17:
   Lys Asp Gln Gln Leu Leu Gly Ile Trp Gly Cys Xaa Gly Xaa Leu Ile
   1               5                   10                  15
   Cys Thr Thr